# EUROPÄISCHE PATENTSCHRIFT

(11) **EP 1 438 576 B1**
(45) Veröffentlichungstag und Bekanntmachung des Hinweises auf die Patenterteilung: **18.02.2009**
(21) Anmeldenummer: 02772244.6
(22) Anmeldetag: 30.08.2002
(51) Int. Cl.: G01N 31/22

(54) **VERFAHREN UND MITTEL ZUR BESTIMMUNG VON GESAMTSÄURE**
METHOD AND MEANS FOR DETERMINING TOTAL ACIDITY
PROCEDE ET AGENT DE DETERMINATION DE L'ACIDITE TOTALE

(30) Priorität: 01.10.2001 DE 10148561
(43) Veröffentlichungstag der Anmeldung: 21.07.2004
(73) Patentinhaber: Merck Patent GmbH, 64293 Darmstadt (DE)
(72) Erfinder: TANZER, Dieter, 64380 Rossdorf (DE); KRATSCHMER, Uwe, 64747 Breuberg (DE)
(86) Internationale Anmeldenummer: PCT/EP2002/009715
(87) Internationale Veröffentlichungsnummer: WO 2003/029810

(56) Entgegenhaltungen:
- EP-A- 0 184 672
- EP-A- 1 248 106
- SU-A- 1 097 946
- US-A- 2 953 439
- US-A- 3 811 837
- PATENT ABSTRACTS OF JAPAN vol. 013, no. 316 (P-900), 18. Juli 1989 (1989-07-18) & JP 01 086063 A (TOYO ROSHI KK), 30. März 1989 (1989-03-30)

## Beschreibung

Die Erfindung betrifft ein trockenchemisches Verfahren und einen Test-Kit zum schnellen, quantitativen Nachweis von Gesamtsäure in Wein und Most.

Wein zertifizierter Herkunft und Qualität muß bestimmte Anforderungen erfüllen. Bevor ein solcher Wein in den Verkehr gebracht werden kann, wird er von spezialisierten Labors mit vorgeschriebenen Analyseverfahren untersucht. Neben dieser Qualitätskontrolle am Ende des Herstellungsprozesses muß der Weinbauer zwischen Traubenernte und Abfüllung des Weines regelmäßig einige wichtige Weinparameter überprüfen, um den Reifeprozess zu steuern, Geschmacksfehler und Präzipitationen zu verhindern, kurzum die gewünschte Qualität zu erreichen.

Säuren sind neben Wasser und Zuckern die dritte Hauptkomponente eines Traubensaftes oder Mostes. Hauptsäuren eines gesunden Traubensaftes sind die in Gramm-Mengen vorliegende Äpfelsäure (Malat) und Weinsäure (Tartrat). Daneben kommen noch eine Vielzahl weiterer Säuren, wie z.B. Zitronensäure (Citrat) und Bernsteinsäure (Succinat) in deutlich geringeren Mengen vor. Die Gesamtsäure stellt die Summe aller titrierbaren Säuren dar. Kohlensäure ist in der Gesamtsäure nicht inbegriffen.

In Mosten kann die Gesamtsäure starken Schwankungen unterliegen. In deutschen Mosten liegt sie, sorten- und reifeabhängig, meist zwischen 9 u. 20 g/l. Zwischen der Zunahme des Zuckergehaltes und der Abnahme des Säuregehaltes während der Traubenreifung besteht dabei ein direkter Zusammenhang. Der Gesamtsäuregehalt nimmt während der Fermentation kontinuierlich ab.

Da der Gesamtsäuregehalt einen wesentlichen Einfluß auf die Geschmacksharmonie eines Weines nimmt, ist der Weinbauer bestrebt, durch geeignete Behandlungsmaßnahmen (z.B: biologischer oder chemischer Säureabbau) einen in Abhängigkeit von der Weinsorte optimalen Säuregehalt (meist ca. 5 - 9 g/l) einzustellen. In weniger guten Jahren enthalten die Moste vor allem in nördlicheren Weinbauländern wie Deutschland mehr Säure als für die Geschmacksharmonie des Weines wünschenswert ist. Umgekehrt darf der Gesamtsäuregehalt teilweise vorgeschriebene Grenzwerte nicht unterschreiten. So muß z.B. Tafelwein einen Gesamtsäuregehalt von mindestens 4,5 g/l aufweisen (Würdig G. und Woller R.: Chemie des Weines (1989) Ulmer-Verlag, Stuttgart; Bergner K.-G.und Lemperle E.: Weinkompendium (1998), Hirzel-Verlag, Stuttgart; Schmidt A.: Aktuelle Weinanalytik (1983), Heller-Verlag, Schwäbisch-Hall).

Der Gesamtsäuregehalt ist deshalb eine für den Kellermeister sehr wichtige Kontrollgröße, die während des Weinausbaues regelmäßig, schnell und zuverlässig und noch möglichst direkt im Weinkeller bestimmt werden muß.

Gemäß der Referenzmethode der EG wird der Gesamtsäuregehalt durch potentiometrische Titration mit einer alkalischen Maßlösung gegen pH 7 ermittelt. Außerhalb der EG (z.B. in den USA und in Australien) wird hingegen eine Titration mit einer alkalischen Maßlösung gegen pH 8,2 vorgeschrieben. Die gebräuchlichen auch von Kellermeistern eingesetzten Methoden bestimmen den Endpunkt nicht potentiometrisch, sondern setzen zur Endpunktsbestimmung Bromthymolblau als Indikator ein (Würdig G. und Woller R.: Chemie des Weines (1989) Ulmer-Verlag, Stuttgart; Bergner K.-G.und Lemperle E.: Weinkompendium (1998), Hirzel-Verlag, Stuttgart; Koch J.: Getränkebeurteilung (1986), Ulmer-Verlag, Stuttgart; Amtsblatt der Europäischen Gemeinschaften, Rechtsvorschriften, 1990). Letztere Verfahrensweise mit visueller Endpunktsbestimmung läßt sich vereinfachen, wenn die zur Titration verwendete Lauge bereits mit Bromthymolblau eingefärbt wird (Blaulauge). Käufliche Titrationslösungen und auf die Titration abgestimmte Titriereinrichtungen erleichtern dem Kellermeister die Bestimmung.

Die Durchführung ist jedoch nach wie vor, insbesondere für Personen ohne Laborerfahrung, umständlich und erfüllt nicht die Ansprüche, die man an eine einfache Bestimmungsmethode stellt. Das Erkennen des Titrationsendpunktes bereitet, besonders bei Rotweinen aufgrund deren Eigenfärbung, große Schwierigkeiten. So überrascht es nicht, daß zwischen der potentiometrischen und der visuellen Methode unterschiedliche Ergebnisse erhalten werden (Lucan Z.D. und Palic A., Die Nahrung 38 (1994), 427 - 433). Der Verbrauch an Titrationsmittel (Blaulauge) ist zudem nicht unerheblich (ca. 10 - 20 ml pro Titration) und zieht Entsorgungsprobleme nach sich.

In jüngerer Zeit hat die Analyse mit festen, saugfähigen Trägern, den sogenannten Teststäbchen, zunehmend auch in der Weinanalytik an Bedeutung gewonnen (Unger-Heumann M. und Tanzer D., G.I.T. Laboratory Journal 3 (1998), 174 -175). Zu den wesentlichen Vorteilen dieser trockenchemischen Verfahren gehören insbesondere die einfache Handhabung sowie die aufgrund der geringen Reagenzmengen unproblematische Entsorgung. Alle oder der größte Teil der für die Nachweisreaktion notwendigen Reagenzien sind dabei in entsprechenden Schichten eines festen, saugfähigen oder quellbaren Trägers eingebettet, auf den die Probe aufgebracht wird. Nach Kontakt der Reaktionszone mit der Probe läuft die Nachweisreaktion ab. Die gebildete Farbe ist ein Maß für die Menge des zu bestimmenden Analyts und kann visuell, d.h. halbquantitativ bzw. quantitativ mit einfachen Reflektometern ausgewertet werden.

Ein trockenchemisches Verfahren zur Bestimmung der Gesamtsäure konnte jedoch bislang nicht realisiert werden.

Der vorliegenden Erfindung liegt daher die Aufgabe zu Grunde, ein Verfahren zur Bestimmung von Gesamtsäure in Most und Wein zur Verfügung zu stellen, das einfach und schnell in der Ausführung, damit auch im Weinkeller einsetzbar, und kostengünstig ist. Insbesondere sollte das erfindungsgemäße Verfahren nicht nur einer halbquantitativen, visuellen, sondern auch einer quantitativen Auswertung mit einem Reflektometer zugänglich sein.

Es wurde gefunden, dass ein einfaches und schnelles trockenchemisches Verfahren zur Bestimmung der Gesamtsäure in Wein und Most zur Verfügung gestellt werden kann, indem die Probe zunächst mit einer leicht alkalischen Pufferlösung versetzt wird und die daraus resultierende Verschiebung des pH-Wertes mit einem Teststäbchen, auf den ein geeigneter pH-Indikator aufgebracht ist, nachgewiesen wird.

Gegenstand der vorliegenden Erfindung ist eine Verfahren zur Bestimmung von Gesamtsäure in Most und Wein, gekennzeichnet durch folgende Verfahrensschritte:
a) Bereitstellen eines Teststäbchens, das zwei Zonen mit unterschiedlichen pH-Indikatoren aufweist;
b) Zugabe eines Tris(hydroxymethyl)-aminomethan-Puffers mit einem pH-Wert im Bereich von 9-12,5 zu der zu analysierenden Probe;
c) Eintauchen des Teststäbchens in das Gemisch aus Schritt b)
d) Auswertung der Farbreaktion auf dem Teststäbchen

In einer bevorzugten Ausführungsform wird in Schritt a) ein Teststäbchen bereit gestellt, das einen oder mehrere der pH-Indikatoren Bromkresolgrün, Phenolrot, Bromphenolrot, Chlorphenolrot und Curcumin enthält.

In einer bevorzugten Ausführungsform erfolgt die Auswertung in Schritt d) reflektometrisch.

Gegenstand der vorliegenden Erfindung ist auch ein Test-Kit zur Bestimmung von Gesamtsäure in Most und Wein zumindest aufweisend einen Tris(hydroxymethyl)-aminomethan-Puffer mit einem pH-Wert im Bereich von 9-12,5 und ein Teststäbchen, das zwei Zonen mit unterschiedlichen pH-Indikatoren aufweist.

In einer bevorzugten Ausführungsform enthält der Test-Kit ein Teststäbchen, das einen oder mehrere der pH-Indikatoren Bromkresolgrün, Phenolrot, Bromphenolrot, Chlorphenolrot und Curcumin aufweist.

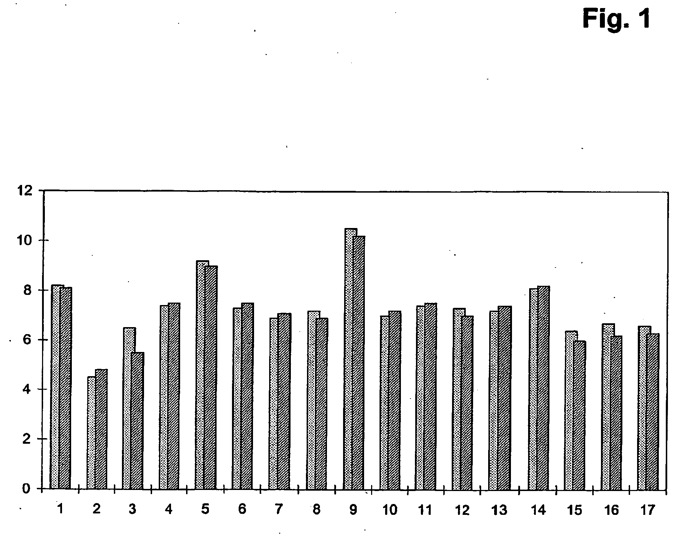

Abbildung 1 ist in Beispiel 2 näher erläutert.

Zur Durchführung des erfindungsgemäßen Verfahrens zur Bestimmung von Gesamtsäure in Most und Wein wird die zu analysierende Probe zunächst mit einer bestimmten Menge Reagenzlösung versetzt. In die Mischung bestehend aus Most bzw. Wein und Reagenzlösung wird dann ein Teststäbchen kurz eingetaucht und die resultierende Farbreaktion auf dem Teststäbchen quantitativ mit Hilfe eines Reflektometers oder halbquantitativ durch Vergleich mit einer Farbkarte ausgewertet. Bei Zugabe der schwach alkalischen Reagenzlösung reagieren die Säuren in der Probe mit der Reagenzlösung unter Verschiebung des pH-Wertes. Diese Veränderung, die dem Säuregehalt proportional ist, wird durch pH-Indikatoren auf dem Teststäbchen nachgewiesen.

Most und Wein bedeutet erfindungsgemäß jede wässrige Probe, die zu einem überwiegenden Teil aus unvergorenem, teilvergorenem oder vergorenem Traubensaft besteht, bevorzugt Traubensaft oder Most, während des Weinausbaus auftretender teilvergorener Most oder Wein. Für den Fachmann ist es jedoch offensichtlich, dass mit Hilfe des erfindungsgemäßen Verfahrens bei entsprechender Kalibration der Gesamtsäuregehalt auch in anderen Säften oder in daraus gewonnenen vergorenen Produkten bestimmt werden kann. Beispiele derartiger Probenmaterialien sind Obst- oder Gemüsesäfte, Obstweine oder Destillate von vergorenen Produkten.

Bei der alkalischen Reagenzlösung, die der Probe zugesetzt wird, handelt es sich um Tris(hydroxymethyl)aminomethanpuffer (TRIS-Puffer) der nach Zugabe zu einer definierten Menge an Probelösung eine gut meßbare pH-Wert-Verschiebung bewirkt. Der pH-Wert der Pufferlösung muß im Bereich 9 -12,5, besonders bevorzugt im Bereich 9,5 -10,5 liegen. Die Konzentration der Pufferlösung muß im Bereich von ca. 1 mmol/L -100 mmol/L, besonders bevorzugt im Bereich 10-50 mmol liegen.

Durch gezielte Abstimmung der Konzentration des Puffers, der Menge an zugegebener Pufferlösung und der Menge an Probelösung läßt sich in Abhängigkeit des Gesamtsäuregehaltes eine pH-Wert-Änderung erzielen, die mittels Testsstäbchen sehr zuverlässig ausgewertet werden kann.

Der pH-Wert eines Weines bzw. Mostes liegt im Bereich von ca. 3. Die Dosierung von Pufferlösung ist so zu bemessen, daß bei Proben mit hohen Gesamtsäuregehalten eine nur leichte, bei Proben mit kleinen Gesamtsäuregehalten eine starke pH-Verschiebung erfolgt, die mit Teststreifen gut ausgewertet werden kann. Der pH-Wert sollte dabei in den Bereich zwischen pH 4 und pH 12, bevorzugt zwischen pH 4 und pH 10 verschoben werden.

Bei der Verwendung des TRIS-Puffers eignet sich z.B. eine Lösung von 2,4 g Tris(hydroxymethyl)-aminomethan auf 1 l Wasser zur Bestimmung von Gesamtsäuregehalten zwischen 1 und 14 g/l, wenn 0,2 ml einer Probe 1 ml der Pufferlösung zugesetzt wird. Liegt der Gesamtsäuregehalt der Probe über 14 g/l, so sollte bei gleicher Probenmenge die zugegebene Menge an Pufferlösung erhöht (Faktor 1,2 - 2,0) werden oder ein etwas stärker konzentrierter Puffer (anstelle 0,02 mol/l, z.B. 0,05 mol/l) verwendet werden bzw. die Menge an Probenmenge verringert werden (z.B. 0,1 ml Probe).

Als Indikatoren auf dem Teststäbchen kommen prinzipiell alle Farbindikatoren bzw. Mischungen von Farbindikatoren in Frage, deren spektrale Eigenschaften im zu untersuchenden pH-Bereich von ca. 4 -12, bevorzugt 4-10, eine deutliche pH-Abhängigkeit aufweisen und sich visuell bzw. mittels eines Reflektometers quantitativ auswerten lassen. Als besonders vorteilhaft für die erfindungsgemäße Bestimmung haben sich die Indikatoren Bromkresolgrün, Phenolrot, Bromphenolrot, Chlorphenolrot und Curcumin und dabei insbesondere Mischungen von zwei oder mehreren dieser Indikatoren erwiesen. Durch Kombination von Nachweiszonen mit unterschiedlichen Farbindikatoren, wie bei gängigen pH-Universalindikatorpapieren üblich, läßt sich die Nachweisempfindlichkeit weiter verbessern.

Die Indikatoren befinden sich auf dem Teststreifen typischerweise in einem saugfähigen Träger, in den auch alle weiteren für den Nachweis notwendigen Reagenzien (z.B. Stabilisatoren und Lösungsvermittler) eingebettet sind. Zumeist bedeckt der saugfähige Träger, auf den die Nachweisreagenzien aufgebracht sind, nicht den gesamten Teststreifen, sondern lediglich eine Zone des Teststäbchens. Auf diese Weise ist es möglich, nicht nur eine Zone mit einer Zusammensetzung von Nachweisreagenzien, sondern mehrere Zonen mit unterschiedlichen Zusammensetzungen, z.B. zum Nachweis verschiedener Konzentrationsbereiche, auf einem Teststäbchen zu kombinieren.

Als saugfähige Träger können alle Materialien verwendet werden, die üblicherweise für solche Tests im Gebrauch sind. Am weitesten verbreitet ist die Verwendung von Filterpapier, jedoch können auch andere saugfähige Cellulose- oder Kunststoffprodukte eingesetzt werden.

Die saugfähigen Träger werden in bekannter Weise mit Tränklösungen imprägniert, die die gewünschten Indikatoren, gegebenenfalls Stabilisatoren und Lösungsvermittler enthalten. Die getränkten und getrockneten Papiere können geeignet zugeschnitten und in bekannter Weise auf Trägerfolien aufgeklebt bzw. aufgesiegelt werden.

Gegenstand der vorliegenden Erfindung ist weiterhin auch ein Test-Kit, der zumindest ein Teststäbchen mit zwei Zonen mit unterschiedlichen pH-Indikatoren enthält, das zur Durchführung des erfindungsgemäßen Verfahrens geeignet ist, und mindestens einen Tris(hydroxymethyl)-aminomethan-Puffer mit einem pH-Wert im Bereich von 9-12,5. Geeignete Teststäbchen sind solche, die Farbindikatoren bzw. Mischungen von Farbindikatoren aufweisen, deren spektrale Eigenschaften im zu untersuchenden pH-Bereich von ca. 4 - 12, bevorzugt 4-10, eine deutliche pH-Abhängigkeit aufweisen und sich visuell bzw. mittels eines Reflektometers quantitativ auswerten lassen.

Von wesentlichem Vorteil für ein Testsystem ist seine lange Lagerfähigkeit ohne Änderung der Testeigenschaften (insbesondere Kalibrationsdaten). Lagerversuche mit den erfindungsgemäßen Testsystem zeigen, daß die angestrebte Lagerzeit von mindestens 2 Jahren Haltbarkeit unter Raumtemperaturbedingungen problemlos erreicht werden kann.

Somit bietet das erfindungsgemäße Verfahren und der erfindungsgemäße Test-Kit erstmals die Möglichkeit, den Gesamtsäuregehalt von Wein oder Most mittels eines einfachen trockenchemischen Verfahrens zu bestimmen. Durch die erfindungsgemäße Zugabe einer schwach alkalischen Pufferlösung zu der Probe wird der pH-Wert der Probe in Abhängigkeit vom Säuregehalt in einen mit dem pH-Indikator-Teststäbchen leicht und zuverlässig meßbaren pH-Bereich verschoben. Die Genauigkeit und Empfindlichkeit des erfindungsgemäßen Verfahrens ist vergleichbar mit der wesentlich aufwendigeren potentiometrischen Titration.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

### Beispiele

### Beispiel 1

### Bestimmung von Gesamtsäure mit Teststäbchen - reflektometrische Auswertung der Reaktionsfarbe:

### Herstellung der Teststäbchen:

Auf Filterpapiere (Firma Binzer, 1450 CV; säuregewaschen) werden nachfolgende Tränklösungen aufgebracht und danach mit warmer Luft getrocknet. Die Papiere werden mit Schmelzkleber (z.B. Kleber Dynapol^{®} 1272) auf eine weiße Trägerfolie aufgesiegelt und geeignet in Streifen geschnitten, so daß Reaktionszonen von ca. 6 mm x 8 mm resultieren.

### Zusammensetzungen der Tränklösungen:

### - Für Meßbereich Gesamtsäure ca. 1 - 8 g/1:

In einen 1L Erlenmeyerkolben werden nacheinander jeweils 0,1%ige alkoholische Lösungen von Bromphenolrot (22 g), Phenolrot (87 g), Curcumin (130 g) und 260 g Ethanol eingewogen.

### - Für Meßbereich Gesamtsäure ca. 7 -14 g/l:

In einen 1L Erlenmeyerkolben werden jeweils 0,1%ige alkoholische Lösungen von Bromkresolgrün (125 g) und Chlorphenolrot (375 g) eingewogen.

### Herstellung der Pufferlösung:

0,24 g Tris(hydroxymethyl)-aminomethan werden unter Rühren in 100 ml vollentsalztes Wasser (VE-Wasser) gelöst.

### Analyse:

Zu 0,2 ml Probe (Wein bzw. Most) wird 1 ml der Pufferlösung gegeben.

Die Teststäbchen werden für die Bestimmung kurzzeitig in die Probelösung eingetaucht, so daß sie vollständig benetzt werden. In Abhängigkeit vom Gesamtsäuregehalt der Probe und dem resultierenden pH-Wert verfärben sich die Farbindikatoren innerhalb weniger Sekunden. Zur quantitativen Auswertung werden die Teststreifen nach 15 Sekunden in einem kleinen Handreflektometer auf Diodenbasis (Reflektometer RQflex^{®}, Merck KGaA, Darmstadt (Gromes R. Tanzer D., G.I.T. 5 (2001), 2 - 5)) ausgewertet. Den Zusammenhang zwischen den gemessenen relativen Remissionen (%) und dem Gesamtsäuregehalt zeigen die Tabellen 1 und 2.

Der Gesamtsäuregehalt der eingesetzten Proben wird mit der EG-Referenzmethode (potentiometrische Titration) ermittelt.

**Tabelle 1**

| Meßbereich Gesamtsäure ca. 1 - 8 g/l: | |
|---|---|
| **Gesamtsäure [g/L]** | **% rel. Remission** |
| 1 | 26 |
| 2 | 32 |
| 3 | 39 |
| 4 | 47 |
| 5 | 55 |
| 6 | 64 |
| 7 | 73 |
| 8 | 81 |

**Tabelle 2**

| Meßbereich Gesamtsäure ca. 7 - 14 g/l: | |
|---|---|
| **Gesamtsäure [g/L]** | **% rel. Remission** |
| 7 | 11 |
| 8 | 23 |
| 9 | 33 |
| 10 | 42 |
| 11 | 50 |
| 12 | 58 |
| 13 | 65 |
| 14 | 72 |

### Beispiel 2:

### Praxistest: Bestimmung von Gesamtsäure in Most- u. Weinproben im Vergleich mit dem Referenzverfahren.

Die Nachweiszonen aus Beispiel 1 werden auf ein Teststäbchen aufgebracht. Der Abstand der beiden 8 x 6 mm großen Testzonen beträgt 4 mm.

Gemäß der Durchführung aus Beispiel 1 werden verschiedene Proben im Vergleich mit dem Referenzverfahren untersucht, wobei der Zusammenhang zwischen Remission und Gesamtsäure aus Beispiel 1 als Kalibrationskurve verwendet wird.

Das Reflektometer RQflex^{®} besitzt eine für diese Art der Auswertung notwendige Doppeloptik.

Die Ergebnisse sind in Abbildung 1 dargestellt. Auf der y-Achse ist der Gesamtsäuregehalt in g/l, auf der x-Achse die Probennummer aufgetragen. Die für die jeweilige Probennummer links aufgetragenen, gepunkteten Balken zeigen das Analyseergebnis der jeweiligen Probe, das mit dem erfindungsgemäßen Verfahren ermittelt wurde. Die für die jeweilige Probe rechts angeordneten gestreiften Balken zeigen das Analyseergebnis, das mit dem Referenzverfahren ermittelt wurde.

## Patentansprüche

1. Verfahren zur Bestimmung von Gesamtsäure in Most und Wein, **gekennzeichnet durch** folgende Verfahrensschritte:
a) Bereitstellen eines Teststäbchens, das zwei Zonen mit unterschiedlichen pH-Indikatoren aufweist;
b) Zugabe eines Tris(hydroxymethyl)-aminomethanpuffers mit einem pH-Wert im Bereich von 9-12,5 zu der zu analysierenden Probe;
c) Eintauchen des Teststäbchens in das Gemisch aus Schritt b)
d) Auswertung der Farbreaktion auf dem Teststäbchen

2. Verfahren nach Anspruch 1, **dadurch gekennzeichnet, dass** in Schritt a) ein Teststäbchen bereit gestellt wird, das als pH-Indikator einen oder mehrere der Indikatoren Bromkresolgrün, Phenolrot, Bromphenolrot, Chlorphenolrot und Curcumin enthält.

3. Verfahren nach Anspruch 1 oder 2, **dadurch gekennzeichnet, dass** die Auswertung in Schritt d) reflektometrisch erfolgt.

4. Test-Kit zur Bestimmung von Gesamtsäure in Most und Wein zumindest aufweisend einen Tris(hydroxymethyl)-aminomethanpuffer mit einem pH-Wert im Bereich von 9-12,5 und ein Teststäbchen, das zwei Zonen mit unterschiedlichen pH-Indikatoren aufweist.

5. Test-Kit nach Anspruch 4, **dadurch gekennzeichnet, dass** der Test-Kit ein Teststäbchen enthält, das als pH-Indikator einen oder mehrere der Indikatoren Bromkresolgrün, Phenolrot, Bromphenolrot, Chlorphenolrot und Curcumin aufweist.

## Claims

1. Method for the determination of total acid in must and wine, **characterised by** the following method steps:
a) provision of a test stick which has two zones with different pH indicators;
b) addition of a tris(hydroxymethyl)aminomethane buffer having a pH in the range 9 - 12.5 to the sample to be analysed;
c) dipping of the test stick into the mixture from step b);
d) evaluation of the colour reaction on the test stick.

2. Method according to Claim 1, **characterised in that** a test stick which comprises, as pH indicator, one or more of the indicators bromocresol green, phenol red, bromophenol red, chlorophenol red and curcumin is provided in step a).

3. Method according to Claim 1 or 2, **characterised in that** the evaluation in step d) is carried out reflectometrically.

4. Test kit for the determination of total acid in must and wine at least comprising a tris(hydroxymethyl)aminomethane buffer having a pH in the range 9 - 12.5 and a test stick which has two zones with different pH indicators.

5. Test kit according to Claim 4, **characterised in that** the test kit comprises a test stick which has, as pH indicator, one or more of the indicators bromocresol green, phenol red, bromophenol red, chlorophenol red and curcumin.

## Revendications

1. Méthode de détermination de l'acide total dans du moût et du vin, **caractérisée par** les étapes méthodologiques suivantes :
a) la fourniture d'un bâtonnet de test comportant deux zones ayant des indicateurs de pH différents ;
b) l'addition d'un tampon tris(hydroxyméthyl)aminométhane ayant un pH dans la plage 9 - 12,5 à l'échantillon à analyser ;
c) l'immersion du bâtonnet de test dans le mélange issu de l'étape b) ;
d) l'évaluation de la réaction colorée sur le bâtonnet de test.

2. Méthode selon la revendication 1, **caractérisée en ce qu'**un bâtonnet de test comprenant, comme indicateur de pH, un ou plusieurs parmi les indicateurs : vert de bromocrésol, rouge de phénol, rouge de bromophénol, rouge de chlorophénol et curcumine, est fourni dans l'étape a).

3. Méthode selon la revendication 1 ou 2, **caractérisée en ce que** l'évaluation dans l'étape d) est effectuée par réflectométrie.

4. Kit de test pour la détermination de l'acide total dans du moût et du vin, comprenant au moins un tampon tris(hydroxyméthyl)aminométhane ayant un pH dans la plage 9-12,5 et un bâtonnet de test comportant deux zones ayant des indicateurs de pH différents.

5. Kit de test selon la revendication 4, **caractérisé en ce que** le kit de test comprend un bâtonnet de test qui possède, comme indicateur de pH, un ou plusieurs parmi les indicateurs : vert de bromocrésol, rouge de phénol, rouge de bromophénol, rouge de chlorophénol et curcumine.
